# EUROPEAN PATENT APPLICATION

(11) **EP 4 394 371 A1**
(43) Date of publication of application: **03.07.2024**
(21) Application number: 22217264.5
(22) Date of filing: 30.12.2022
(51) Int. Cl.: G01N 27/414, C12Q 1/00, G01N 33/48

(54) **A BIO-FET-BASED PEPTIDE SEQUENCING METHOD AND DEVICE**

(71) Applicant: Imec VZW, 3001 Leuven (BE)
(72) Inventor: Martens, Koen, 9880 Aalter (BE); Van Helleputte, Nick, 3060 Korbeek-Dijle (BE); Van Dorpe, Pol, 3510 Spalbeek (BE); Santermans, Sybren, 3560 Lummen (BE)
(74) Representative: Roth, Sebastian

(57) **Abstract**

The present disclosure relates to single-peptide sequencing. A method for sequencing peptides attaches a peptide (15) with one end to a channel region surface (13) of a biosensor field effect transistor (bio-FET, 10) and obtains a first measurement of a parameter of the bio-FET, which depends on its threshold voltage. The method further provides first molecular probes (16) into the electrolyte gate (14), each first molecular probe being linked to a charge tag (17), and obtains second measurements of the parameter. Then, the method determines whether a shift of the parameter occurred between the first and second measurements, which indicates that a first molecular probe attached to an amino acid at a free end of the peptide. If the shift of the parameter occurred, the method determines a type of the amino acid based on a type of the first molecular probe.

## Description

### TECHNICAL FIELD

The present disclosure relates to peptide sequencing, in particular, to single-peptide sequencing. The disclosure provides a method and a biosensor device for sequencing peptides. The disclosure also provides a biosensor field effect transistor (bio-FET), which can be used in the method and the biosensor device. The peptide sequencing with the bio-FET uses molecular probes that can bind to amino acids of the peptide, wherein the molecular probes are linked to charge tags.

### BACKGROUND

Conventional single-molecule proteomics implemented on chip sensors suffer from a lack of throughput. The lack of throughput is especially a culprit for proteomics, because proteomics typically investigate a protein or peptide population, in which different types of molecules have concentrations that are different by orders of magnitude. For sequencing, this requires reading out billions of single molecules.

An exemplary technique (the SeqLL technique) is currently used for DNA sequencing, and is able to scan one base (to identify four different nucleobase types) of one billion different single molecules in about one hour. Given that proteins may have 20 types of amino acid building blocks, and prototypical peptides (protein fragments, which can be used to identify the original protein) may be composed on average of 30 amino acids, this exemplary technique would result in very long sequencing times of the proteins or peptides. In particular, sequencing times of 150 hours or more, if the technique were re-purposed for the latest proteomics on chip techniques.

### SUMMARY

In view of the above, this disclosure aims for an improved technique for sequencing peptides.

An objective is to provide a method and biosensor device for sequencing peptides, which achieves a higher throughput than the conventional techniques. Thus, an objective is to reduce the time needed for sequencing peptides. The method and biosensor device should accordingly enable parallel peptide sequencing. Another objective is to provide an on chip solution.

These and other objectives are achieved by the solutions of the independent claims. Advantageous implementations are described in the dependent claims.

A first aspect of this disclosure provides a method for sequencing peptides, the method comprising: providing a peptide to attach with one end to a channel region surface of a biosensor field effect transistor (bio-FET) having an electrolyte gate; obtaining a first measurement of a parameter of the bio-FET that depends on a threshold voltage of the bio-FET; providing one or more first molecular probes into the electrolyte gate, wherein each first molecular probe is linked to a charge tag; obtaining one or more second measurements of the parameter of the bio-FET; determining whether a shift of the parameter occurred between the first measurement and one of the second measurements, which indicates that one of the first molecular probes attached to an amino acid at a free end of the peptide; and if the shift of the parameter between the first measurement and one of the second measurements occurred, determining a type of the amino acid at the free end of the peptide based on a type of the first molecular probes.

With the method of the first aspect, a single peptide may be sequenced using the bio-FET. The method can also sequence multiple peptides individually and in parallel, for instance, by using multiple bio-FETs. The sequencing of the one or more peptides can be implemented on chip, wherein the chip may include one or more of the bio-FETs.

With the method of the first aspect, a time needed for sequencing the one or more peptides can be reduced, and a throughput of sequencing peptides can thus be significantly increased.

The parameter that depends on the threshold voltage of the bio-FET may be the threshold voltage of the bio-FET itself. However, the parameter could also be a current through the bio-FET, or any other threshold voltage dependent parameter of the bio-FET.

The method of the first aspect may also determine whether a shift of the parameter occurred between any of the measurements performed, for example, in order to also account for a drift of the parameter, which is not related to the attachment of the molecular probe.

The charge tag may have a certain size, such that when the first molecular probe attaches to the peptide, the charge tag is arranged close (e.g., within 1-3 nanometers) to the channel region surface of the bio-FET or even touches the channel region surface of the bio-FET. This boosts the shift of the parameter, which occurs when the molecular probe attaches to the peptide.

In an implementation, the method further comprises: providing one or more second molecular probes into the electrolyte gate, wherein each second molecular probe is linked to a charge tag; obtaining one or more third measurements of the parameter of the bio-FET; determining whether a shift of the parameter occurred between the first or one of the second measurements and one of the third measurements, which indicates that one of the second molecular probes attached to the amino acid at the free end of the peptide; and if the shift of parameter between the first or one of the second measurements and one of the third measurements occurred, determining the type of the amino acid at the free end of the peptide based on a type of the second molecular probes.

For example, the above described measurement cycle with the second molecular probes may be performed, if the shift of the parameter of the bio-FET between the first measurement and the one or more second measurements did not occur. In another example, it may be desired to perform the measurement cycle with the second molecular probes notwithstanding a negative or positive result in the previous measurement cycle with the first molecular probes. This may be useful, because certain molecular probes may not be specific enough to determine a single type of amino acid, and the measurement cycle with the second molecular probes may give more insight as to the type of the amino acid at the free end of the peptide. It may also be desired to repeat a measurement cycle with a certain type of molecular probes one or more times, in order to confirm the previously obtained results.

After a shift of the parameter has been detected, it may also be beneficial to remove the charge tag linked to a molecular probe. The charge tags are large, and this could make it more difficult for another charge tag to attach. The charge tag can be removed, for example, by at least one of photo-cleavage, chemical cleavage, change of pH, and change of charge, and by flushing away.

In an implementation, the method further comprises: degrading the peptide, wherein the amino acid at the second end of the peptide is removed; obtaining a fourth measurement of the parameter of the bio-FET; providing a plurality of third molecular probes into the electrolyte gate, wherein each third molecular probe is linked to a charge tag; obtaining one or more fifth measurements of the parameter of the bio-FET; determining whether a shift of the parameter occurred between the fourth measurement and one of the fifth measurements, which indicates that one of the third molecular probes attached to an amino acid at a free end of the degraded peptide; if the shift of the parameter between the fourth measurement and one of the fifth measurements occurred, determining a type of the amino acid at the free end of the degraded peptide based on a type of the third molecular probes.

For example, this measurement cycle with the third molecular probes may be performed, if the shift of the parameter of the bio-FET between the first measurement and one of the second measurements or one of the third measurements occurred. However, it may also be desired to observe several positive results in measurement cycles with different molecular probes, in order to identify the amino acid. Thus, more measurement cycles may be performed, particularly, more than the three specific measurement cycles described above.

In an implementation of the method, if any molecular probe attaches to the amino acid at the free end of the peptide or of the degraded peptide, a distance between the charge tag linked to the attached molecular probe and the channel region surface of the bio-FET is in a range of 3 nm or less, or in a range of 1 nm or less.

The small distance between the charge tag and the channel region surface may ensure that the shift of the measured parameter of the bio-FET is sufficiently pronounced. This is achieved, because due to the small distance the threshold voltage of the bio-FET changes sufficiently, even if only single peptide is sequenced using the bio-FET, i.e., only a single molecular probe linked to a charge tag is able to attach to the peptide. This boost provided by the use of the charge tags enables single-peptide sequencing.

In an implementation, the method further comprises: promoting a binding of the charge tag, which is linked to the attached molecular probe, to the channel region surface of the bio-FET by at least one of: modifying or setting a pH of the electrolyte gate; modifying the charge tag with a reagent; providing additives to the electrolyte to induce binding between the charge tag and/or bio-FET surface; modifying a charge of the charge tag.

For instance, a Cu-catalyzed click can be activated by adding a Cu catalyst as the additive into the electrolyte gate. Promoting the binding of the charge tag may further increase the signal of the bio-FET (i.e., the shift of the parameter of the bio-FET in case the molecular probe is attached to the (single) peptide).

In an implementation, the method comprises, in order to achieve that the respective molecular probes provided into the electrolyte gate are linked to the charge tags: providing the molecular probes into the electrolyte gate, and then providing the charge tags into the electrolyte gate, wherein the charge tags are configured to link to the molecular probes; or providing the molecular probes already linked to the charge tags into the electrolyte gate.

In an implementation of the method, each charge tag has a characteristic size of larger than 10 nm and/or comprises more than 100 elementary charges.

In an implementation, the method further comprises providing a buffer into the electrolyte gate, in order to remove from the electrolyte gate any molecular probes that are not attached to the peptide or to the degraded peptide, before or while obtaining the one or more second, third, or fifth measurements of the parameter of the bio-FET.

In an implementation of the method, the one or more second, third, or fifth measurements of the parameter of the bio-FET are obtained with a measuring frequency in a range of 1-100 kHz.

In an implementation of the method, the steps of the method, which are performed for the bio-FET, are in parallel also performed for one or more other bio-FETs.

In an implementation of the method, all the bio-FETs are arranged in a sensor array, and a number of the bio-FETs in the sensor array is at least 1000.

The fast measurements of the parameter of the bio-FET, and the parallel sequencing by using multiple bio-FETs, enables achieving a significantly increased throughput.

In an implementation of the method, a size of the channel region surface of each bio-FET is in a range of 50 nm or less.

Thus, many of the bio-FETs can be arranged on a chip, wherein the chip may still have a reasonably small area.

A second aspect of this disclosure provides bio-FET comprising: a source and a drain; a channel region arranged between the source and the drain, the channel region having a channel region surface; an electrolyte gate covering at least the channel region surface; a peptide attached to the channel region surface with one end; a molecular probe being attached to an amino acid at a free end of the peptide; herein a charge tag is linked to the attached molecular probe; and wherein a distance between the charge tag and the channel region surface is in a range of 3 nm or less, or is in a range of 1 nm or less.

The bio-FET of the second aspect enables single-peptide sequencing. This is due to the fact that, as in the above-described configuration in which the molecular probe with the charge tag is attached to the peptide, the distance between the charge tag and the channel region surface is small. Therefore, a change of the parameter of the bio-FET compared to a configuration without attached molecular probe, is high enough to be detected.

A third aspect of this disclosure provides a biosensor device for sequencing peptides, the biosensor device comprising: a bio-FET comprising a source, a drain, a channel region having a channel region surface, and an electrolyte gate covering at least the channel region surface; a measurement unit configured to measure a threshold voltage of the bio-FET; a flow controller configured to provide liquid flows to the electrolyte gate; and a processor; wherein, in order to perform the method of the first aspect or any of its implementation forms, the processor is configured to: control the flow controller to provide a liquid flow comprising the peptide into the electrolyte gate; control the measurement unit to obtain the first measurement of the parameter of the bio-FET; control the flow controller to provide a liquid flow including the one or more first molecular probes into the electrolyte gate; control the measurement unit to obtain the one or more second measurements of the parameter of the bio-FET; determine whether a shift of the parameter occurred between the first measurement and one of the second measurements, which indicates that one of the first molecular probes attached to an amino acid at a free end of the peptide; and if the shift of the parameter between the first measurement and one of the second measurements occurred, determine a type of the amino acid at the free end of the peptide based on a type of the first molecular probes.

The biosensor device may be a chip, which may include one or more bio-FETs. Multiple bio-FETs can be grouped into a sensor cell of the biosensor device, wherein the biosensor device can have one or more sensor cells.

In an implementation of the biosensor device, the processor is further configured to: control the flow controller to provide a liquid flow including the one or more second molecular probes into the electrolyte gate, wherein each second molecular probe is linked to a charge tag; control the measurement unit to obtain the one or more third measurements of the parameter of the bio-FET; determine whether a shift of the parameter occurred between the first or one or the second measurements and one of the third measurements, which indicates that one of the second molecular probes attached to the amino acid at the free end of the peptide; and if the shift of the parameter between the first or one of the second measurements and one of the third measurements occurred, determine the type of the amino acid at the free end of the peptide based on a type of the second molecular probes.

The further measurement cycle with this second molecular probes may, for example, be carried out, if the shift of the threshold voltage between the first measurement and the one or more second measurements did not occur, as this may indicate that the first molecular probes cannot attach to the amino acid at the free end of the peptide. However, it is also possible to repeat the measurement cycle with the first molecular probes one or more times, in order to confirm the negative result in the measurement cycle with the first molecular probes, or to carry out the measurement cycle with the second molecular probes even if the result of the measurement cycle with the first molecular probes was positive. The more information is obtained, the more accurate the type of the amino acid at the free end of the peptide may be determined.

The biosensor device of the third aspect achieves the same advantages as the method of the first aspect, and may be extended by respective implementations as described above for the method of the first aspect.

In summary of the above aspects and implementations, the present disclosure addresses the challenge to sequence many (for example, billions of) peptides at single-molecule level with a sufficiently high throughput. The challenge arises mainly because of the fundamentally diffraction-limited sensor density of conventional optical sensors.

A sufficiently high single molecule signal (e.g., larger than 10 mV threshold voltage shift if a molecular probe attaches to the peptide), which allows reading out each bio-FET of a dense bio-FET array with a sense amplifier, can be achieved by introducing the charge tags onto the molecular probes. The different molecular probes are used to identify different amino acids of the peptide. The signal may be further boosted by promoting the surface adherence of the charge tags to the channel region surface.

### BRIEF DESCRIPTION OF THE DRAWINGS

The above described aspects and implementations are further explained in the following description of embodiments with respect to the following drawings:
- FIG. 1: shows a biosensor device for sequencing peptides according to an embodiment of this disclosure, wherein the biosensor device includes a bio-FET.
- FIG. 2: shows a method for sequencing peptides according to an embodiment of this disclosure.
- FIG. 3: illustrates steps of a method according to an embodiment of this disclosure for implementing a peptide sequencing process.

### DETAILED DESCRIPTION OF EMBODIMENTS

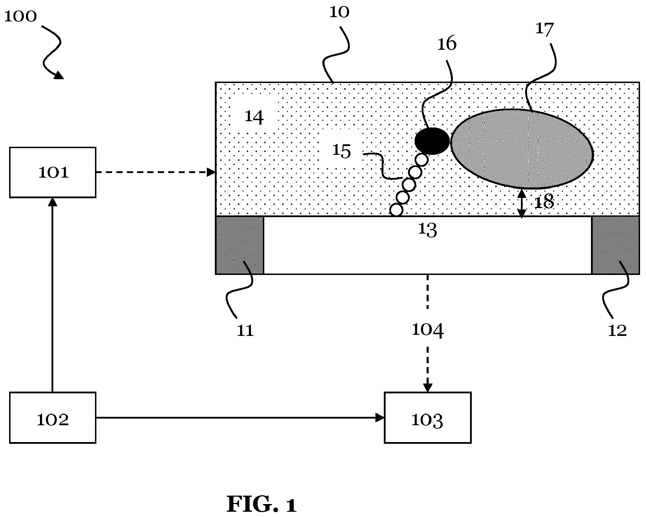

FIG. 1 shows a biosensor device 100 according to an embodiment of this disclosure. The biosensor device 100 is configured to sequence peptides, in particular, the biosensor device 100 supports single-peptide sequencing.

The biosensor device 100 comprises a bio-FET 10, a processor 102, a flow controller 101, and a measurement unit 103. The bio-FET 10 comprises a source 11, a drain 12, a channel region having a channel region surface 13, and an electrolyte gate 14 covering at least the channel region surface 13.

The measurement unit 103 may be a sense amplifier. The measurement unit 103 is configured to measure a parameter of the bio-FET 10 that depends on a threshold voltage of the bio-FET 10. The measurement unit 103 may also measure the threshold voltage of the bio-FET directly as said parameter. The measurement 103 unit may be configured to continuously or repeatedly measure said parameter of the bio-FET 10. For example, the measurement unit 103 may measure the parameter of the bio-FET 10 with a measuring frequency in a range of 1-100 kHz. The measurement unit 103 may be configured to measure the parameter by measuring a bias current of the bio-FET 10, which flows between the source 11 and the drain of the bio-FET 10, for different voltages provided to the channel region surface 13 via the electrolyte gate 14.

The flow controller 101 is configured to provide one or more liquid flows to the electrolyte gate 14, for example, a first liquid flow at a first time point, and a second liquid flow at a second time point. For example, a particular liquid flow provided by the flow controller 101 may comprise a particular type of molecular probes, or a particular type of charge tags, or a particular type of molecular probes already linked each to a particular charge tag. A particular liquid flow may also provide a buffer into the electrolyte gate 14, for example, in order to remove molecular probes and/or charge tags provided previously from the electrolyte gate 14.

The processor 102 may comprise processing circuitry configured to perform, conduct or initiate various operations described in this disclosure. The processing circuitry may comprise hardware and/or the processing circuitry may be controlled by software. The hardware may comprise analog circuitry or digital circuitry, or both analog and digital circuitry. The digital circuitry may comprise components such as application-specific integrated circuits (ASICs), field-programmable arrays (FPGAs), digital signal processors (DSPs), or multi-purpose processors. The processor 102 may further comprise memory circuitry, which stores one or more instruction(s) that can be executed by the processing circuitry, in particular, under control of the software. For instance, the memory circuitry may comprise a non-transitory storage medium storing executable software code which, when executed by processing circuitry, causes the various operations of the processor 102 described in this disclosure to be performed.

In particular, the processor 102 is configured to control the flow controller 101 and the measurement unit 103. The processor 102 is configured to control the flow controller 101 to provide a particular liquid flow into the electrolyte gate 14. The processor 102 is configured to control the measurement unit 103 to obtain one or more measurements 104 of the parameter of the bio-FET 10. The processor 102 is also configured to determine, whether a shift of the parameter occurred between consecutive measurements 104 of the parameter. The processor 102 is also configured to determine a type of an amino acid of the peptide 15 based on a type of a molecular probe that lead to a shift of the parameter of the bio-FET 10.

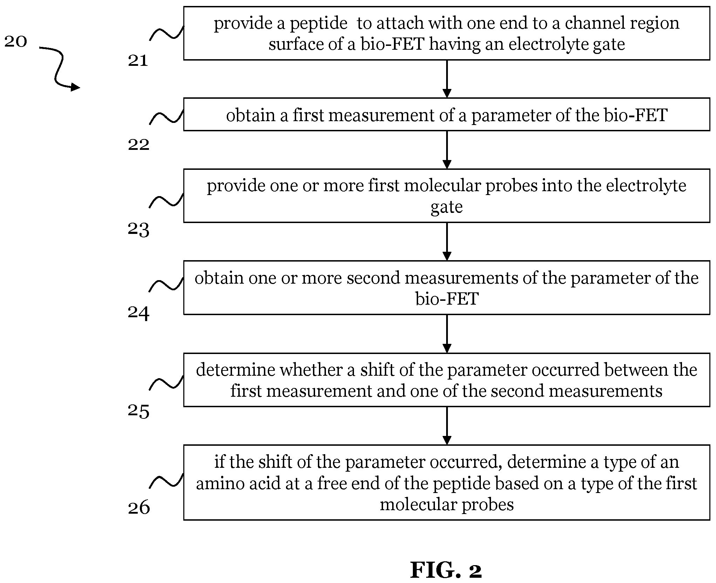

Generally, the processor 102 may be configured to control the biosensor device 100 to perform the method 20, which is shown as a flow-diagram in FIG. 2. The method 20 according to this disclosure is usable for sequencing peptides.

The method 20 comprises a step 21 of providing a peptide 15 to attach with one of its ends to a channel region surface 13 of the bio-FET 10. To implement this method step 21, the processor 102 can control the flow controller 101 to provide a liquid flow comprising the peptide 15 into the electrolyte gate 14.

The method 20 further comprises a step 22 of obtaining a first measurement 104 of the parameter of the bio-FET 10. To implement this method step 22, the processor 102 can control the measurement unit 103 to obtain the first measurement 104.

The method 20 further comprises a step 23 of providing one or more first molecular probes 16 into the electrolyte gate 14, wherein each first molecular probe 16 is linked to a charge tag 17. To implement this method step 23, the processor 102 can control the flow controller 101 to provide a liquid flow including the one or more first molecular probes 16 into the electrolyte gate 14. The first molecular probes 16 may already be linked to charge tags 17. Alternatively, the processor 102 can further control the flow controller 101 to provide a liquid flow including the charge tags 17 into the electrolyte gate 14, wherein the introduced charge tags 17 are then configured to link to the first molecular probes 16 already in the electrolyte gate 14.

The method 20 further comprises a step 24 of obtaining one or more second measurements 104 of the parameter of the bio-FET 10. To implement this method step 24, the processor 102 can control the measurement unit 103 to obtain the second measurements 104.

The method 20 further comprises a step 25 of determining whether a shift of the parameter occurred between the first measurement 104 and one of the second measurements 104. To implement this method step 25, the processor 102 can compare the results of the first and second measurements 104, and to determine whether the respectively measure parameters have different values.

A shift of the parameter may indicate that one of the first molecular probes 16, which is linked to a charge tag 17, has attached to an amino acid at a free end of the peptide 15, as shown in FIG. 1. The peptide 15 is attached to the channel region surface 13 of the bio-FET 10 with the other end. The first molecular probe 16 is attached to the end of the peptide 15 that is not attached to the channel region surface 13 of the bio-FET 10. The charge tag 17 is linked to the attached first molecular probe 16. According to an embodiment of this disclosure, a distance 18 between the charge tag 17 and the channel region surface 13 in this case is in a range of 3 nm or less. In another embodiment, the distance 18 is in a range of 1 nm or less. The charge tag 17 may even touch and/or adhere to the channel region surface 13.

Notably, the bio-FET 10 may be gated by changes in the surface potential of the channel region surface 13, wherein such changes may be induced, for instance, by the charge tag 17 being in close proximity of the channel region surface 13, or even binding to the channel region surface 13. The charge tag 17 may influence or induce a charge distribution of a semiconductor material below the channel region surface 13 (which may be a dielectric surface), and may thus cause a change of the threshold voltage of the bio-FET 10, and also of the parameter, like a current flowing between the source 11 and the drain 12 of the bio-FET 10.

If the shift of the parameter between the first measurement 104 and one of the second measurements 104 occurred, the method 20 further comprises a step 26 of determining a type of the amino acid at the free end of the peptide 15 based on a type of the first molecular probes 16.

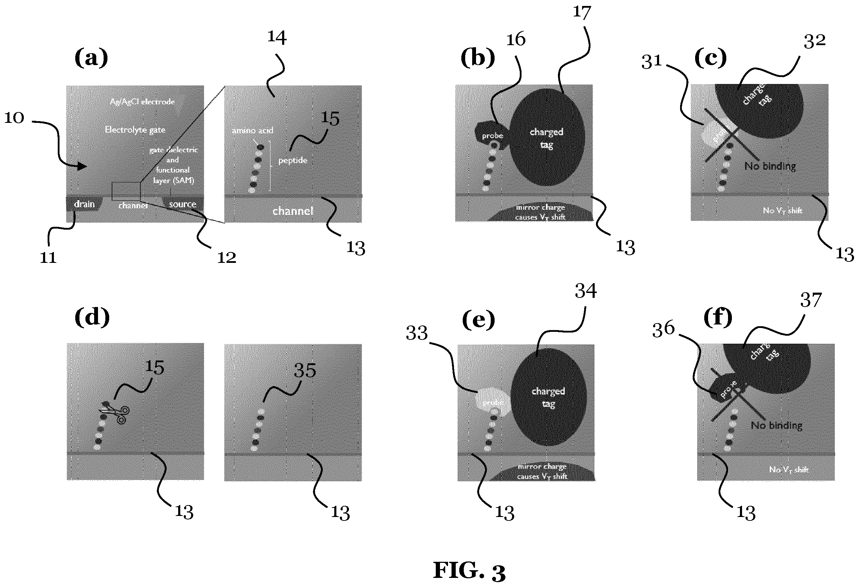

FIG. 3 illustrates several steps of the method 20 according to an embodiment of this disclosure, which builds on the general method 20 shown in FIG. 2. The method 20 illustrated in FIG. 3 may be used for implementing a peptide sequencing process, for example, of a single peptide. The method 20 enables a higher throughput in sequencing one or more peptides than, for example, a conventional optical system used for sequencing. The method 20 may be performed by the biosensor device 100 shown in FIG. 1 or any other chip.

FIG. 3(a) shows a bio-FET 10 of the biosensor device 100 or chip, and shows how the peptide 15 is attached with one end to the channel region surface 13 of the bio-FET 10.

FIG. 3(b) shows a scenario, where first molecular probes 16 are introduced into the electrolyte gate 14. A first molecular probe 16 has attached to the amino acid at the free end of the peptide 15. A charge tag 17 is linked to the first molecular probe 17. Due to its size, the charge tag 17 is close to the channel region surface 13, which leads to a mirror charge being induced in the channel region of the bio-FET 10 below the channel region surface 13, and accordingly causes a shift of the threshold voltage of the bio-FET 10.

Therefore, also the parameter measured by the measurement unit 103 changes. The change of the parameter indicates that the first molecular probe 16 is attached to the peptide 15, and thus allows determining the type of the amino acid at the free end of the peptide 15 according to the type of the first molecular probe 16.

FIG. 3(c) shows a scenario, where second molecular probes 31 are introduced into the electrolyte gate 14. The second molecular probes 31 are linked to charge tags 32. However, no second molecular probe 31 is able to attach to the amino acid at the free end of the peptide 15, for instance, because the second molecular probes 31 are of the wrong type for the type of amino acid at the free end of the peptide 15. Accordingly, no shift of the threshold voltage occurs, and also no change in the parameter can be measured. Consequently, the processor 102 can also not determine a shift of the parameter between a current measurement 104 of the parameter and a previous measurement 104 of the parameter, for example, before the second molecular probes 31 were introduced into the electrolyte gate 14 of the bio-FET.

FIG. 3(d) shows that the peptide 15 may be degraded, which includes that the amino acid at the free end of the peptide 15 is removed. The degrading of the peptide 15 results in the degraded peptide 35, which may have a different type of amino acid at its free end.

FIG. 3(e) shows a scenario, where third molecular probes 33 are introduced into the electrolyte gate 14. The third molecular probes 33 are linked to charge tags 34. However, no third molecular probe 33 is able to attach to the amino acid at the free end of the degraded peptide 35. For instance, because the third molecular probes 33 are of the wrong type for the type of amino acid at the free end of the degraded peptide 35. Accordingly, no shift of the threshold voltage occurs, and also no change of the parameter can be measured. Consequently, the processor 102 can also not determine a shift of the parameter between a current measurement 104 of the parameter and a previous measurement 104 of the parameter, for example, before the third molecular probes 33 were introduced into the electrolyte gate 14 of the bio-FET 10.

FIG. 3(f) shows a scenario, where fourth molecular probes 36 are introduced into the electrolyte gate 14. A fourth molecular probe 36 is attached to the amino acid at the free end of the degraded peptide 35. A charge tag 37 is linked to the fourth molecular probe 36. Due to its size, the charge tag 37 is close to the channel region surface 13, which leads to a mirror charge being induced in the channel region of the bio-FET 10 below the channel region surface 13, and causes a shift of the threshold voltage of the bio-FET 10. Accordingly, also the parameter measured by the measurement unit 103 changes. The change of the parameter indicates that the fourth molecular probe 36 is attached to the peptide 15, and thus may allow determining the type of the amino acid at the free end of the degraded peptide 35 according to the type of the fourth molecular probe 36.

This method 20 can continue further (not shown), until the entire peptide 15 is sequenced, i.e., until a type of each amino acid of the peptide 15 is determined. The method 20 may sequence the peptide 15 faster than a conventional method.

Notably, the first molecular probes 16 maybe identical to the third or the fourth molecular probes 33, 36, and the second molecular probes 31 may be identical to the third or the fourth molecular probes 33, 36 (but different from the first molecular probes 16), respectively. The same holds for the charge tags 17, 32, 34, 37. More than the mentioned molecular probes and/or charge tags may be used in a sequencing process. The use of further molecular probes for further measurements 104 may have different reasons. For instance, if the processor 102 determines that no shift of the threshold voltage dependent parameter occurred so far. But also, if further information and/or confirmation is needed to determine the type of an amino acid at a free end of the peptide 15 or the degraded peptide 35. Molecular probes in this disclosure maybe antibodies, nanobodies, aptamers, or the like.

The method 20 may also make use of multiple nanoscale bio-FETs 10. These bio-FETs 10 may each have a channel region surface 13 with a size in a range of 50 nm or less. Each bio-FET 10 can electrically detect a single molecule, which has docked onto or is in close vicinity to the channel region surface 13. The channel region surface 13 of multiple bio-FETs 10 may be exposed to the sample liquid, i.e., the electrolyte gate 14. The bio-FETs 10 may each have an area that is much smaller than that of an optical sensor device (e.g., of a photodiode in an imager pixel). The bio-FETs 10 can potentially each have an area as small as 0.01 µm² or even smaller, which can be achieved with advanced CMOS technology. Hence, even a larger number of bio-FETs 10, e.g. in the order of billions, could be integrated on a chip of the approximate size in the order of cm² or smaller.

To be able to arrange the plurality of bio-FETs 10 in such arrays of billions, a shift of their threshold voltages, which may be induced by docking a single molecule onto the respective channel region surface 13s, should preferably be in the order of 10-100 mV. Fulfilling this allows a sense amplifier to read out a group of the bio-FETs 10 on the same bit line. To allow such signals (i.e. shifts of threshold voltages), this disclosure proposes dedicated modifications or additions of the molecular probes 16, 31, 33, 36, which are used to identify the amino acids of peptides 15 or degraded peptides 35 as shown above. These molecular probes 16, 31, 33, 36 are typically specific for a single amino acid, and may dock onto their preferred amino acid binding partner, hence, allowing to identify the type of amino acid at the free end of the investigated peptide 15 or degraded peptide 35. A special technique may be used to attach a single peptide 15 (i.e., the peptide 15 to be sequenced) onto the respective channel region surface 13 of each bio-FET 10 of the array.

The above-mentioned modifications or additions to the molecular probes 16, 31, 33, 36, which allow a good electrical detection of the binding to the peptide 15 or degraded peptide 35, include using the charge tags 17, 32, 34, 37. These charge tags may be large, e.g., may have a 10 nm or more characteristic size, and e.g. may each have 100 or more elementary charges. In this disclosure, charge tags 17, 32, 34, 37 may comprise DNA or RNA constructs, proteins, DNA origami, nanobeads, or the like. These charge tags 17, 32, 34, 37 may link to the molecular probes 16, 31, 33, 36, and may help to generate a sufficiently strong electrical signal, e.g., a 10 mV or larger shift of the threshold voltage of the respective bio-FET 10. Such a shift, and a corresponding shift of the measured parameter, is well detectable by the measurement unit 103, which may, for example, be a sense amplifier, and allows the processor 102 to accurately identify the presence of an attached molecular probe 16, 31, 33, 36.

The charge tags 17, 32, 34, 37 may be docked onto the molecular probes 16, 31, 33, 36, after these molecular probes have specifically bound onto their target amino acids of the peptide 15 or the degraded peptide 35 attached to the bio-FET 10. This can, for instance, be realized with click chemistry. For example, by introducing an azide group on the molecular probe 16, 31, 33, 36, such that a DBCO or alkyne group on the charge tag 17, 32, 34, 37 can specifically bind to the azide group of the molecular probe. Different, (e.g. bio-orthogonal) covalent conjugation chemistries can be used for this purpose, as well as non-covalent methods such as, for example, biotin-streptavidin.

To promote the close adherence of the charge tags 17, 32, 34, 37 to the channel region surface 13 of the bio-FET 10, if the molecular probe 16, 31, 33, 36 is attached to the peptide 15, in order to improve the signal, an additional step can be introduced that links the respective charge tag 17, 32, 34, 37 with the channel region surface 13 of the bio-FET 10, after the charge tag-peptide assembly has formed. This could be done, for instance, by changing the environment to promote a binding of the charge tag 17, 32, 34, 37 to the bio-FET's 10 channel region surface 13 with a functional layer. For example, by a pH change to induce hydrogen binding or Coulomb binding (in this case, the reference measurement 104 may be done at the same pH and the amino-acid molecular probe binding may be done at a different pH, wherein the probe-amino acid binding may be sufficiently maintained at the other pH).

Alternatively, the charge tag's 17, 32, 34, 37 affinity for the channel region surface 13 of the bio-FET 10 can be increased by modifying the respective charge tag 17, 32, 34, 37 with a reagent. For instance, by introducing a reagent that renders a surface of the charge tag 17, 32, 34, 37 more hydrophobic for the case of a hydrophobic functional layer on the bio-FET 10. The hydrophobic surfaces will be promoted to join. Alternatively, the charge tag 17, 32, 34, 37 can be rendered from a negative or neutral charge to a positive charge for the case of a negatively charged bio-FET surface 13 (as typical for oxides). The positive charge tag 17, 32, 34, 37 will become attracted to the negative surface 13.

The bio-FETs 10 may be organized in sensor cells of the biosensor device 100, wherein each sensor cell may have as few bio-FETs 10 as possible, to reduce sensor cell area. A minimum is two bio-FETs 10 in a sensor cell, for instance, in a source follower configuration. In this example, the second bio-FET 10 may play the role of selecting the word line to read out a subpopulation of the bio-FETs 10 simultaneously. Apart from the word line selection, the sensor cell may also play the role of a minimized pre-amplifier to lighten the role of the sense amplifier. In the sensor cell, a bio-FET 10 have the electrolytic gate 14 shared by the other bio-FETs 10. The shared electrolyte gate 14 may have a fixed potential applied by a reference electrode, which can be integrated into the chip, or which can be a macroscopic electrode. Other FETs may exist in the sensor cell, and may have a solid gate, and may hence be classical FETs.

The sensor cells of the biosensor device 100 may be organized in arrays, akin to CMOS based memories, in which bit lines are read out by sense amplifiers. Word lines may be used to select which device on a bit line is read out. The sense amplifiers may measure voltage or current due to the selected bio-FET, which is a key difference with typical memories, which are inherently digital and typically work with larger signals.

Nevertheless, the sensor array may detect the presence, or not, of a molecular probe 16, 31, 33, 36 on each of its bio-FETs 10 (in contrast to e.g. a continuous, analog readout value), and hence will have a digital character. The processor 102 of the biosensor device 100 may determine, whether a molecular probe 16, 31, 33, 36 has docked to a peptide 15, by comparing the state of the respective bio-FET 10 before and after the docking chemistry has been applied. This state may be acquired before and after using the same pure buffer, in other words the same electrolyte. Hence prior to applying the docking chemistry, the same buffer may be applied without the docking chemistry to record the reference signal value of the bio-FET 10 (e.g., the first measurement 104). Optionally also after applying the docking chemistry, the buffer could be replaced by a pure buffer to flush away any free floating molecular probes prior to recording the 'after' signal. This relative signal collection may be done, in order to avoid drift and shifts due to buffer changes. This relative digital character of the bio-FET 10 will aid in coping with noise, variability, drift and other bio-FET parasitics. Variability of, e.g., the threshold voltage is inherent in the nanoscale FET devices, and can be coped with in such fashion for bio-FETs 10. Digitization is known to be less susceptible to the impact of noise. Such digitization can be realized by implementing a limited number of digitization signal levels (e.g., 6 bit) to allow the before and after comparison. The signal level steps would be in the order of the size of the signal, or smaller.

The readout frequency, at which the measurement unit 103 (e.g., including one or more sense amplifiers) are operated may be on the order of kHz, in order to allow for the sensitivity and also the throughput of the system. An array of a billion sensor cells read out by 10000 sense amplifiers would be readable with kHz sense amps in 60s. This may allow sequencing a billion 30-amino acid peptides 15 in 10 hours, i.e., significantly faster than an optical system, in which the time bottle neck is due to the optical readout. For the biosensor device 100, an important aspect is the fluidics. The above timing estimate is for the case when for each amino acid 20 molecular probes are consecutively allowed to bind with the target peptide 15. So the total time of 10 hours is calculate by 60s x 20 amino acid probes x 30 amino acids per peptide 15. In the biosensor device 100 fast fluidics may thus be incorporated to carry out the necessary reagent exchanges in the time frames required. This may be achieved by the fluid controller 103 and the processor 102.

Multi-level detection can be introduced to speed up the biosensor device 100 further. In this case, multiple molecular probes 16, 31, 33, 36 may be detected simultaneously. In this case 2ⁿ (where n = 1, 2, 3, ...) different electrical charge tags 17, 32, 34, 37 can be discriminated by device. These charge tags 17, 32, 34, 37 can be chosen to have different charge and/or different size to allow for different signals (i.e., parameter shifts as result of threshold voltage shifts). This would allow to reduce the peptide sequencing time by 2ⁿ, or to reduce reagent binding times by carrying out binding of different molecular probes 16, 31, 33, 36 in parallel.

The method 20 of this disclosure has several advantages. In particular, with the method 20, large single molecule signals (i.e., >10 mV shifts of the threshold voltage of a bio-FET 10) can be obtained with a 10 nm size protein as a charge tag 17, 32, 34, 37, wherein the charge tag has in the order of 100 elementary charges.

In the claims as well as in the description of this disclosure, the word "comprising" does not exclude other elements or steps and the indefinite article "a" or "an" does not exclude a plurality. A single element may fulfill the functions of several entities or items recited in the claims. The mere fact that certain measures are recited in the mutual different dependent claims does not indicate that a combination of these measures cannot be used in an advantageous implementation.

## Claims

1. A method (20) for sequencing peptides, the method (20) comprising:
providing (21) a peptide (15) to attach with one end to a channel region surface (13) of a biosensor field effect transistor, bio-FET, (10) having an electrolyte gate (14);
obtaining (22) a first measurement (104) of a parameter of the bio-FET (10) that depends on a threshold voltage of the bio-FET (10);
providing (23) one or more first molecular probes (16) into the electrolyte gate (14), wherein each first molecular probe (16) is linked to a charge tag (17);
obtaining (24) one or more second measurements (104) of the parameter of the bio-FET (10);
determining (25) whether a shift of the parameter occurred between the first measurement (104) and one of the second measurements (104), which indicates that one of the first molecular probes (16) attached to an amino acid at a free end of the peptide (15); and
if the shift of the parameter between the first measurement (104) and one of the second measurements (104) occurred, determining (26) a type of the amino acid at the free end of the peptide (15) based on a type of the first molecular probes (16).

2. The method (20) according to claim 1, further comprising:
providing one or more second molecular probes (31) into the electrolyte gate (14), wherein each second molecular probe (31) is linked to a charge tag (32);
obtaining one or more third measurements (104) of the parameter of the bio-FET (10);
determining whether a shift of the parameter occurred between the first or one of the second measurements (104) and one of the third measurements (104), which indicates that one of the second molecular probes (31) attached to the amino acid at the free end of the peptide (15); and
if the shift of the parameter between the first or one of the second measurements (104) and one of the third measurements (104) occurred, determining the type of the amino acid at the free end of the peptide (15) based on a type of the second molecular probes (31).

3. The method (20) according to claim 1 or 2, further comprising:
degrading the peptide (15), wherein the amino acid at the free end of the peptide (15) is removed;
obtaining a fourth measurement (104) of the parameter of the bio-FET (10);
providing a plurality of third molecular probes (33) into the electrolyte gate (14), wherein each third molecular probe (33) is linked to a charge tag (34);
obtaining one or more fifth measurements (104) of the parameter of the bio-FET (10);
determining whether a shift of the parameter occurred between the fourth measurement (104) and one of the fifth measurements (104), which indicates that one of the third molecular probes (33) attached to an amino acid at a free end of the degraded peptide (35);
if the shift of the parameter between the fourth measurement (104) and one of the fifth measurements (104) occurred, determining a type of the amino acid at the free end of the degraded peptide (35) based on a type of the third molecular probes (33).

4. The method (20) according to one of the claims 1 to 3, wherein if any molecular probe (16, 31, 33, 36) attaches to the amino acid at the free end of the peptide (15) or of the degraded peptide (35), a distance (18) between the charge tag (17, 32, 34, 37) linked to the attached molecular probe (16, 31, 33, 36) and the channel region surface (13) of the bio-FET (10) is in a range of 3 nm or less, or in a range of 1 nm or less.

5. The method (20) according to one of the claims 1 to 4, further comprising:
promoting a binding of the charge tag (17, 32, 34, 37), which is linked to the attached molecular probe (16, 31, 33, 36), to the channel region surface (13) of the bio-FET (10) by at least one of:
- modifying or setting a pH of the electrolyte gate (14);
- modifying the charge tag (17, 32, 34, 37) with a reagent;
- providing additives to the electrolyte gate (14) to induce binding between the charge tag (17, 32, 34, 37) and the channel region surface (13);
- modifying a charge of the charge tag (17, 32, 34, 37).

6. The method (20) according to one of the claims 1 to 5, wherein the method (20) comprises, in order to achieve that the respective molecular probes (16, 31, 33, 36) provided into the electrolyte gate (14) are linked to the charge tags (17, 32, 34, 37):
providing the molecular probes (16, 31, 33, 36) into the electrolyte gate (14), and then providing the charge tags (17, 32, 34, 37) into the electrolyte gate (14), wherein the charge tags (17, 32, 34, 37) are configured to link to the molecular probes (16, 31, 33, 36); or
providing the molecular probes (16, 31, 33, 36) already linked to the charge tags (17, 32, 34, 37) into the electrolyte gate (14).

7. The method (20) according to one of the claims 1 to 6, wherein each charge tag (17, 32, 34, 37) has a characteristic size of larger than 10 nm and/or comprises more than 100 elementary charges.

8. The method (20) according to one of the claims 1 to 7, further comprising:
providing a buffer into the electrolyte gate (14), in order to remove from the electrolyte gate (14) any molecular probes (16, 31, 33, 36) that are not attached to the peptide (15) or to the degraded peptide (35), before or while obtaining the one or more second, third, or fifth measurements (104) of the parameter of the bio-FET (10).

9. The method (20) according to one of the claims 1 to 8, wherein the one or more second, third, or fifth measurements (104) of the parameter of the bio-FET (10) are obtained with a measuring frequency in a range of 1-100 kHz.

10. The method (20) according to one of the claims 1 to 9, wherein the steps of the method (20), which are performed for the bio-FET (10), are in parallel also performed for one or more other bio-FETs (10).

11. The method (20) according to claim 10, wherein all the bio-FETs (10) are arranged in a sensor array, and a number of the bio-FETs (20) in the sensor array is at least 1000.

12. The method (20) according to one of the claims 1 to 11, wherein a size of the channel region surface (13) of each bio-FET (10) is in a range of 50 nm or less.

13. A biosensor field effect transistor, bio-FET, (10) comprising:
a source (11) and a drain (12);
a channel region arranged between the source (11) and the drain (12), the channel region having a channel region surface (13);
an electrolyte gate (14) covering at least the channel region surface (13);
a peptide (15) attached to the channel region surface (13) with one end;
a molecular probe (16, 33) being attached to an amino acid at a free end of the peptide (15);
wherein a charge tag (17, 34) is linked to the attached molecular probe (16, 33); and
wherein a distance (18) between the charge tag (17, 34) and the channel region surface (13) is in a range of 3 nm or less, or is in a range of 1 nm or less.

14. A biosensor device (100) for sequencing peptides, the biosensor device (100) comprising:
a bio-FET (10) comprising a source (11), a drain (12), a channel region having a channel region surface (13), and an electrolyte gate (14) covering at least the channel region surface (13);
a measurement unit (103) configured to measure a parameter of the bio-FET (10) that depends on a threshold voltage of the bio-FET (10);
a flow controller (101) configured to provide liquid flows to the electrolyte gate (14); and
a processor (102);
wherein, in order to perform the method (20) of one of the claims 1 to 12, the processor (102) is configured to:
control the flow controller (101) to provide a liquid flow comprising the peptide (15) into the electrolyte gate (14);
control the measurement unit (103) to obtain the first measurement (104) of the parameter of the bio-FET (10);
control the flow controller (101) to provide a liquid flow including the one or more first molecular probes (16) into the electrolyte gate (14);
control the measurement unit (103) to obtain the one or more second measurements (104) of the parameter of the bio-FET (10);
determine whether a shift of the parameter occurred between the first measurement (104) and one of the second measurements (104), which indicates that one of the first molecular probes (16) attached to an amino acid at a free end of the peptide (15); and
if the shift of the parameter between the first measurement (104) and one of the second measurements (104) occurred, determine a type of the amino acid at the free end of the peptide (15) based on a type of the first molecular probes (16).

15. The biosensor device (100) according to claim 14, wherein the processor (102) is further configured to:
control the flow controller (101) to provide a liquid flow including the one or more second molecular probes (31) into the electrolyte gate (14), wherein each second molecular probe (31) is linked to a charge tag (32);
control the measurement unit (103) to obtain the one or more third measurements (104) of the parameter of the bio-FET (10);
determine whether a shift of the parameter occurred between the first or one or the second measurements (104) and one of the third measurements (104), which indicates that one of the second molecular probes (31) attached to the amino acid at the free end of the peptide (15); and
if the shift of the parameter between the first or one of the second measurements (104) and one of the third measurements (104) occurred, determine the type of the amino acid at the free end of the peptide (15) based on a type of the second molecular probes (31).
